# EUROPEAN PATENT APPLICATION

(11) **EP 0 558 290 A2**
(43) Date of publication of application: **01.09.1993**
(21) Application number: 93301357.5
(22) Date of filing: 24.02.1993
(51) Int. Cl.: C12Q 1/68, C12N 15/10

(54) **An improved differential screening protocol that allows the detection of marginally induced or repressed mRNAs**

(30) Priority: 28.02.1992 US 843398
(71) Applicant: ELI LILLY AND COMPANY, Indianapolis, Indiana 46285 (US)
(72) Inventor: Lemke, Stephanie Jean, Indianapolis, Indiana 46236 (US); Moore, Robert Earl, Jr., Indianapolis, Indiana 46256 (US)
(74) Representative: Hudson, Christopher Mark

(57) **Abstract**

The invention differential screening technique, single plate one transfer (SPOT), which allows for the sensitive and reliable detection of mRNAs induced or repressed only 2 to 3 fold or less with respect to background. The basis for this technique is to symmetrically spot in quadruplicate or more on a single plate, a large number of potentially positive plaques obtained from a primary, conventional screen. Only one lift is made from this plate in order that there will be minimal variability in DNA transfer. This filter is cut into symmetrical sections such that DNA from all plaques are multiply represented on each section. The sections are next hybridized with different probes. Since each section contains an approximately identical amount of DNA per plaque, one may accurately detect mRNAs that are induced or repressed only slightly above background.

## Description

Differential cloning is a valuable technique which has yielded significant insight into the molecular mechanisms of action of a variety of agents. See e.g., St. John, T. P. and R. W. Davis (1979) Cell 16:443-452; Sambrook J., E. F. Fritsch and T. Maniatis, (1989) Molecular Cloning: A Laboratory Manual, Second Edition, Cold Spring Harbor Laboratory, Cold Spring Harbor, NY. In contrast to subtractive cloning, which primarily detects the new appearance or total disappearance of a given mRNA, differential cloning measures the induction (or repression) in the level of a given mRNA from a basal state. Subtractive cloning is not able to detect such differentially regulated mRNA species. To fully understand the effect of an external stress on mRNA levels, it is essential that one be able to detect and quantify mRNA species which are expressed at different levels in the organism in both treated and untreated states (i.e. "differentially regulated").

Many abundant proteins, such as structural proteins, require the production of large quantities of the corresponding mRNAs. mRNAs for these highly expressed proteins are therefore readily detectable by conventional techniques. However, many "scarce" mRNAs, present only in a relatively few copies per cell, are often important regulatory factors. For example, Alberts, et al. demonstrate the abundance of these relatively "scarce" mRNAs in the following manner:

| The Population of mRNA Molecules in a Typical Mammalian Cell | | | |
|---|---|---|---|
| Class of mRNA | Copies/cell of each mRNA species | Number of different mRNAs in each class | Total # of mRNAs in each class |
| Abundant | 12000 | 4 | 48,000 |
| Intermediate | 300 | 500 | 150,000 |
| Scarce | 15 | 11,000 | 165,000 |

Molecular Biology of the Cell, 2d. Ed.(1989) Garland Publishing, New York, Table 9-2, page 528. While the author's use of the terms "abundant", "intermediate" and "scarce" are somewhat arbitrary, the profusion of these "scarce" mRNA species implicates their indispensable role in the survival of the cell. L. Klickstein points out that,
"A potential drawback to the differential screening approach is that rare sequences will have very low specific probe concentrations in the mixture and thus might not hybridize to the DNA from a target plaque in a reasonable time (overnight)." Current Protocols in Molecular Biology, Ausubel, et al., Eds.(1989 and supplements) John Wiley and Sons, Supplement 4, p.5.8.11.

The value of conventional differential cloning is dimished by its insensitivity and its ability to detect only relatively abundant mRNAs. Thus, the differential regulation of such scarce mRNAs is difficult (if not impossible) to detect by conventional differential cloning techniques. A more sensitive system to allow detection of mRNAs induced or repressed at low levels with respect to background was needed.

The instant invention addresses the shortcomings of existing differential cloning techniques by providing an improved technique to detect mRNAs which are induced or repressed at low levels with respect to background, is easy to perform, and provides a verifiable method to insure the plaque is a true positive. This technique, hereinafter referred to as "SPOT", an abbreviation for "Single Plate, One Transfer", allows one to routinely and accurately detect and plaque purify clones which are induced or repressed only about 2 to 3 fold, or less, with respect to background levels of mRNA. This invention extends the sensitivity and reproducibility of differential cloning.

The attached figures are merely illustrative of the technique of the present invention and should not be considered as limiting on the scope of the claimed invention in any way.

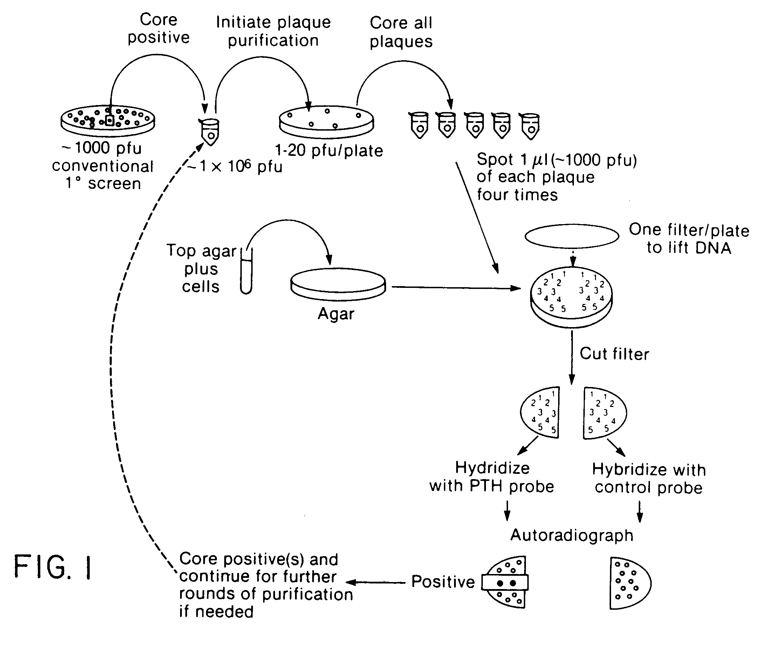

Figure 1 -- Schematic representation of SPOT, (Single Plate One Transfer). This figure depicts a single potentially positive plaque, identified from a conventional, primary screen, being cored from a plate of approximately 1,000 pfu (plaque forming units). This plaque is then diluted and plated to give approximately 1-20 pfu/plate. The technique may be repeated until a particular clone is purified.

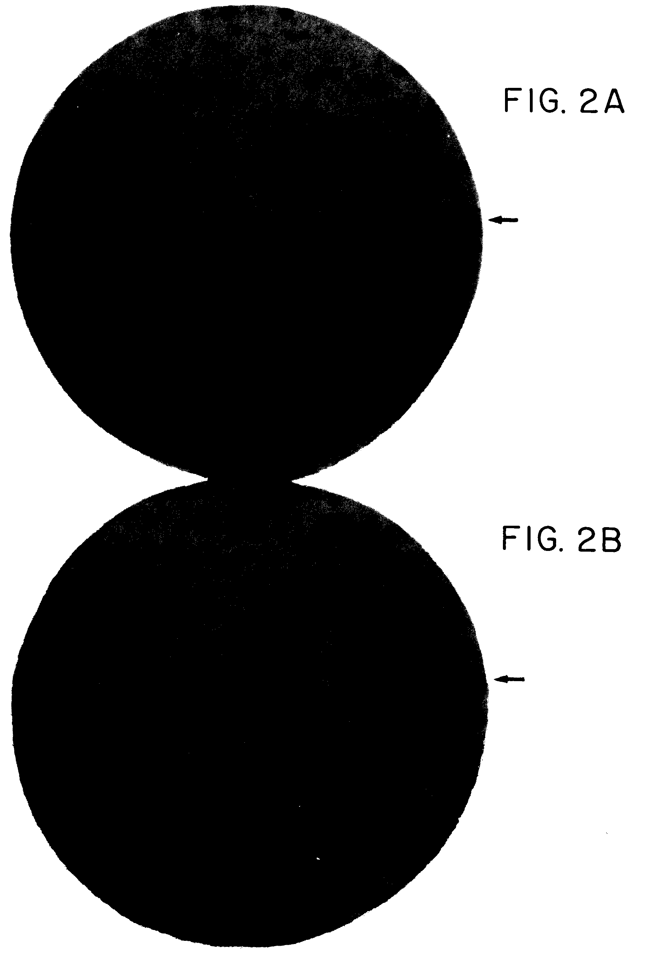

Figure 2 -- An autoradiogram from a conventional, first round screen using differential probes. The plate from which these filters were lifted contained approximately 1,000 pfu. Shown is one of the duplicate filters hybridized with a treated probe (i.e. derived from parathyroid hormone (PTH) treated cells) (A) or untreated probe (B). The arrows point to the differentially regulated plaque.

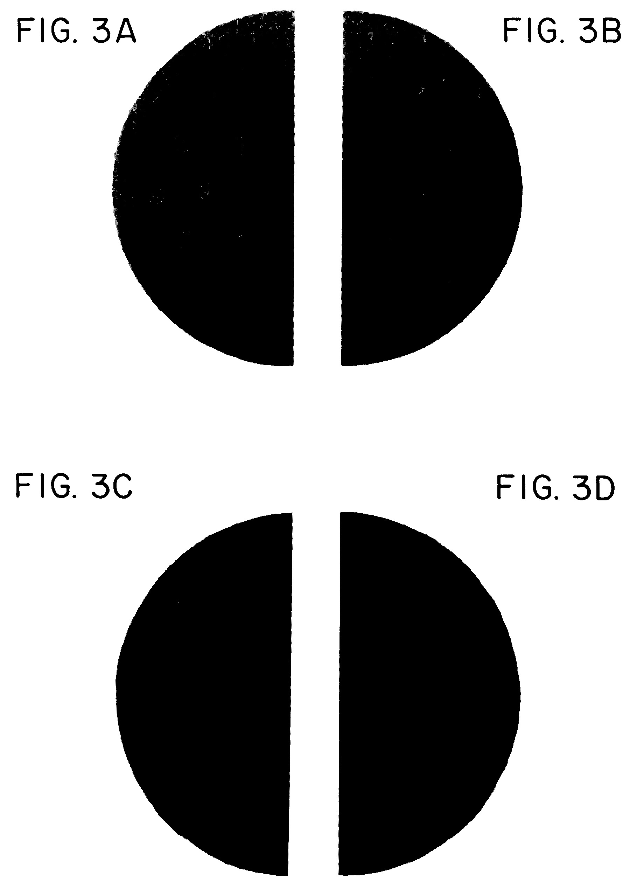

Figure 3 -- An autoradiogram from a second round screen utilizing SPOT. An isolated first round positive plaque was cored and plated out. Subsequently, all of these plaques were cored. Shown are five of these plaques (and a positive control, marked POS). Each plaque was serially spotted in quadruplicate across one plate. A single filter lift was performed and the filter was cut in half. The left half (A) was hybridized with PTH-treated probe and the right half (B) was hybridized with untreated probe. Three of the five clones (#3-5) were up-regulated in response to PTH. Filter halves were stripped of their probe and rehybridized with the opposite probe. The left half (C) was now hybridized with untreated probe and the right half (D) hybridized with PTH-treated probe. The hybridization pattern for #3-5 was reversed, confirming the up-regulation of the pre-pro-alpha-2 chain of type I collagen mRNA.

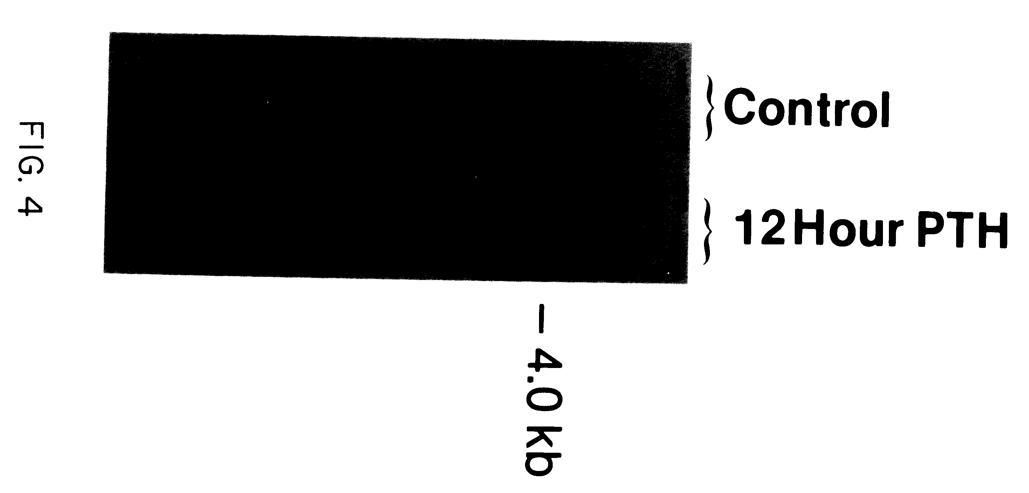

Figure 4 -- Northern blot of mRNA encoding the pre-pro-alpha-2 chain of type I collagen. 10 µg per lane of 12 hour PTH-treated and untreated rat osteosarcoma 17/2.8 mRNA was loaded in duplicate and electrophoresed on a 1% formaldehyde gel in substantial accordance with the teaching of Starling, et al, (1990) Cancer Research 50:7634-7640. Following electrophoresis, the gel was blotted and hybridized with purified, random-primed DNA from the treated clone. The blot was washed at 60°C for one hour in 0.1 x SSPE(0.18 M NaCl, 10mM NaH₂PO₄, 1mM EDTA, pH7.4)/0.1% SDS and exposed to film overnight at -70°C. The pre-pro-alpha-2 chain of type I collagen mRNA was measured to be 4 kb in comparison to size markers run on the same gel. Sequencing of the differentially regulated cDNA clone confirmed the corresponding message to be pre-pro-alpha-2 chain of type I collagen. This figure confirms that the type I pre-procollagen mRNA is induced by parathyroid hormone.

Differential cloning is used to determine the change in number of a given mRNA species in response to some stimulus or environmental stress. A comparison is made between the mRNA species present in the "treated" (i.e. exposed to some environmental stress) versus the untreated state. The increase or decrease of a particular mRNA species in response to some stimulus indicates that the stimulus is either an inducer or repressor of mRNA levels respectively. Conventional differential cloning comprises the steps of: (a) isolating mRNA from treated and untreated cells, (b) constructing a cDNA library from the isolated mRNAs, (c) plating out the cDNA library, (d) culturing the cells to produce multiple plaques on the media, (e) lifting DNA from these plaques onto multiple filter papers so as to produce a replica of the pattern of the plaques on the media, and (f) hybridizing these filters with either treated or untreated probes. By comparing the hybridization pattern revealed in the autoradiograms of the filters from treated and untreated probes one may reveal those plaques which potentially indicate the presence or absence of an induced or repressed mRNA species. The potential positive plaques are then replated onto new plates to be reprobed. The procedure is repeated until positive clones are purified.

A limitation of the conventional differential cloning technique described above is that it lacks sufficient sensitivity to study mRNAs induced or repressed to a minor extent. For example, in order to identify PTH-induced or repressed (parathyroid hormone-induced or repressed) mRNAs, an attempt was made to identify the presence of mRNA induced or repressed in response to the presence of PTH via conventional differential cloning techniques. See e.g., Sambrook, et al., Molecular Cloning, A Laboratory Manual, (1989) Cold Spring Harbor Laboratory, Cold Spring Harbor, NY. In an attempt to decrease the variability of this approach, four lifts were taken from each plate. Each duplicate lift was probed with either treated or untreated probes. During the studies it was observed that the first filter lift removes the majority of the DNA. Each successive lift removes a lesser but more equivalent quantity of DNA. Therefore, the number of filter lifts was increased to five lifts, discarding the first lift of each plate and probing the remaining four filters. Despite this modification, many of the potentially positive clones were false positives. In one experiment, seven potentially induced clones were isolated after screening a total of approximately 20,000 phage on 20 plates. Of these seven potential positives, none were able to be confirmed as true positives by conventional techniques. Later, it was found that one clone was indeed a true positive as later determined by the SPOT technique. Furthermore, for the second round of screening, induced signals were undetectable over background.

We therefore developed the method of the present invention, said method comprising the steps of:
a) identifying a plaque or plaques which contain(s) a potentially differentially regulated mRNA species by conventional differential cloning techniques,
(b) obtaining a sample of a said plaque(s),
(c) serially spotting said sample of said plaque(s) across a solid media surface,
(d) lifting, and fixing DNA from said solid media surface onto a transfer membrane,
(e) dividing said transfer membrane such that each section contains a sample of DNA from each serially spotted plaque,
(f) exposing one section of said transfer membrane to treated probes and the other section of said transfer membrane to untreated probes, and
(g) identifying induced or repressed mRNA by the pattern of differential hybridization.

A schematic representation of this preferred practice of this technique as exemplified herein is shown in Figure 1 of the accompanying drawings. By the method of the present invention, hereinafter known as "SPOT" (Single Plate One Transfer), after the first round of screening, one utilizes only 1 filter lift to examine each group of potential, first round positives as opposed to the use of multiple filter lifts for each potentially positive plaque. A significant advantage of this method is that each plaque transfers an approximately equal amount of DNA to the same filter, eliminating much of the variability in screening by conventional techniques. Furthermore, the SPOT technique allows one to analyze a number of different phage on one filter. Two other aspects of this approach contribute significantly to increase the overall sensitivity. First, the phage are spotted individually onto a plate and are grown to a much larger size than would typically be possible in conventional screening. This allows larger amounts of DNA to be transferred to filters, giving a more reproducible signal that develops in a short period of time. Secondly, phage are spotted serially across the plate giving a number of comparable plaques for every initial plaque. This is in contrast to conventional screening in which: (a) only one signal per plaque is possible, (b) the single plaque is not grown to as large a size, (c) one needs to reprobe the same filter a number of times, and/or (d) perform multiple filter lifts with corresponding variability in the quantity of DNA on different membranes. The combined variability of these factors prevents detection of marginally induced or repressed mRNAs.

Conventional differential cloning protocols are described in readily available laboratory manuals in the art such as Sambrook J., E. F. Fritsch and T. Maniatis, (1989) Molecular Cloning: A Laboratory Manual, Second Edition, Cold Spring Harbor Laboratory, Cold Spring Harbor, NY or Current Protocols in Molecular biology, and Supplements, Ausubel, et al Eds.,(1989 and supplements) John Wiley and Sons, NY.

By "obtaining a sample of" indicates that one may pick up a quantity of the cells by a variety of means. In the preferred practice of the invention as exemplified herein, the plaque is "cored". By "coring" as the term is used herein is to pierce a plaque of interest with tip of a sterile pasteur pipette so as to transfer a sample of the phage into the tip of the pasteur pipette.

By "serially spotting" as the term is used herein means to pipette an equal amount of the sample onto the surface of an agar plate in a manner so as to achieve a symmetrical pattern across the plate. For example, by serially spotting the cored plaques of interest linearlly across a conventional agar plate four times, one may perpendicularly divide the transfer membrane from the replica plating in a manner so as produce two halves of the transfer membrane which are roughly symmetrical. See Figure 3 for an illustration. Although, as exemplified herein, each phage pool was spotted 4 times (2 per section), there is no reason one could not spot the phage a greater number of times to extend the sensitivity of the technique even further. By the same reasoning, one could also divided the transfer membrane into numerous sections and hybridize them with a larger number of different probes.

Following a single lift, the transfer membrane is cut into sections and hybridized with treated or untreated probes. Nearly any material onto which DNA may be transferred and fixed for probing is acceptable as a tranfer membrane. Examples of commonly used acceptable transfer membranes include nitrocellulose filter paper, nylon membranes, velvet cloth, cheesecloth and the like. In the preferred practice of the invention as exemplified herein, the transfer membrane is a nylon membrane such as an Amersham Hybond^{TM} Membrane.

A "treated probe" as used herein denotes a probe capable of identifying the presence of the induced or repressed form of the mRNA. In the preferred practice of the invention as exemplified herein, "treated probes" were radiolabelled complementary DNA sequences produced in the following manner: (a) mRNA is isolated from treated cells, (b) the mRNA is copied into a pool of unlabelled cDNA, and (c) an aliquot of the unlabelled cDNA is copied into labelled DNA with the use of random primers in the presence of P³²-labelled nucleotides to produce the desired P³²-labelled probe. An "untreated probe" as used herein denotes a probe capable of identifying the presence of the untreated form of the mRNA. In the preferred practice of the invention as exemplified herein, untreated probes were radiolabelled complementary DNA sequences produced in the following manner: (a) mRNA is isolated from untreated cells, (b) the mRNA is copied into a pool of unlabelled cDNA, and (c) an aliquot of the unlabelled cDNA is copied into labelled DNA with the use of random primers in the presence of P³²-labelled nucleotides to produce the desired P³²-labelled probe.

Identification of the hybridization pattern is achieved by autoradiography of the transfer membrane sections on X-ray film. Protocols for appropriate conditions for autoradiography are well known in the art See e.g. Sambrook, et al. supra. The transfer media containing the replica plating of the phage onto the media surface (containing the serially spotted potential positives) is divided. One section is exposed to the treated probe and and a second section is exposed to the untreated probe. A comparison of X-ray signals from treated versus untreated probes readily identifies messages which are differentially regulated. It should be noted that the great majority of plaques give the same intensity of signal with both probes, and, as expected, are not differentially regulated.

The methods of the instant invention may be repeated until the plaque of interest is purified and can be characterized. As exemplified herein, following a conventional first round screen, two rounds of SPOT were required to purify the plaque of interest.

The foregoing presumes that one is incorporating the cDNA library into a lambda derived viral vector. However, the use of a plasmid based vector system is equally acceptable. When using plasmid based vectors the method of the present invention comprises the steps of:
a) identifying a colony or colonies which contain(s) a potentially differentially regulated mRNA species by conventional differential cloning techniques,
(b) obtaining a sample of a said colony(s),
(c) serially spotting said sample of said colony(s) across a solid media surface,
(d) lifting and fixing DNA from said solid media surface onto a transfer membrane,
(e) dividing said transfer membrane such that each section contains DNA from each serially spotted colony,
(f) exposing one section of said transfer membrane to treated probes and the other section of said transfer membrane to untreated probes, and
(g) identifying induced or repressed mRNA by the pattern of differential hybridization.

The advantages and elements of the practice of the invention are the same as that previously discussed with respect to the viral based vector system.

The invention further provides a method to confirm induction of mRNA in either a plasmid or viral based vector, said method comprising the steps a-g above, further comprising the steps of:
(h) removing the treated or untreated probe from individual sections,
(i) rehybridizing the section previously exposed with the treated probe with the untreated probe and rehybridizing the section previously exposed to untreated probe with the treated probe, and
(j) evaluating these sections for a reversal of the previous hybridization pattern.

Following hybridization and analysis, filter sections may be freed of probe and rehybridized with the opposite probe, e.g. the untreated versus the treated probe and vice versa. Reversal of the pattern of hybridization confirms that the induction or repression of the mRNA is real. Figure 2 shows an example of a differentially induced clone isolated from our conventional, primary screen. The autoradiograms reproduced in Figures 3A and 3B show this clone as analyzed by SPOT technique. The autoradiograms of Figure 3C and 3D show the result when filters are stripped of probe and rehybridized with the opposite probe to give a reversed hybridization pattern to confirm the differential regulation of the message.

The present invention also includes a kit of materials necessary for the practice of the instant invention. A variety of kits useful for the practice of biotechnology processes are available in the marketplace. Similarly, a kit comprising materials for differential cloning, transfer membranes, culture vessels and media could be assembled and marketed to facilitate the practice of the instant invention. Such a kit could further comprise treated and/or untreated probes to detect the presence of particular mRNA species.

The SPOT technique, as described above and in the following examples, was used to identify and then verify the induction of a PTH induced message from ROS 17/2.8 cells as the pre-pro-alpha-2 chain of type one collagen. Sequencing of the DNA was accomplished using an Applied Biosystems (Foster City, CA) Model 380A or 380B automated DNA sequencer in substantial accordance with the instructions provided by the manufacturer.

To accurately measure the level of induction of the mRNA, Northern Blots and densitometry were performed. As exemplified herein, approximately 10 µg per lane of 12 hour PTH-treated and untreated rat osteosarcoma 17/2.8 mRNA was loaded in duplicate and electrophoresed on a 1% formaldehyde gel in substantial accordance with the teaching of Starling, et al, (1990) Cancer Research 50:7634-7640. Following electrophoresis, the gel was blotted and hybridized with purified random-primed DNA from our induced clone. The blot was washed at 60°C for one hour in 0.1 x SSPE/0.1% SDS to remove unbound probe and exposed to film overnight at -70°C. The pre-pro-alpha-2 chain of type I collagen mRNA was measured to be 4 kb in comparison to size markers run on the same gel. Figure 4 of the accompanying drawings illustrates the results of a Northern blot which confirms that the isolated plaque does indeed encode a corresponding induced mRNA of 4 kb. The identity of the message was confirmed by DNA sequencing. Sequencing of the DNA was accomplished using an Applied Biosystems (Foster City, CA) Model 380A or 380B automated DNA sequencer in substantial accordance with the instructions provided by the manufacturer. Densitometric scanning of this gel demonstrated that the mRNA was induced only 2.5 fold over background.

The art teaches that the administration of PTH to a number of different rat osteosarcoma cell lines in vitro results in a down regulation of both the mRNA and the protein for pre-pro-alpha-2 chain of type I collagen. Kream, et al. (1989) Hormonal Regulation of Collagen Gene Expression in Osteoblastic Cells: Overview and New Findings, Connective Tissue Research 20:187- 192; Kream, et al. (1980) Parathyroid Hormone Alters Collagen Synthesis and Procollagen mRNA Levels in Fetal Rat Calvaria, Proc. Natl. Acad. Sci. 77:5654-5658; Partridge, et al. (1989) Parathyroid Hormone Inhibits Collagen Synthesis at Both Ribonucleic Acid and Protein Levels in Rat Osteogenic Sarcoma Cells, Mol. Endocrinology 3:232-239. The results demonstrated herein clearly differ from previously reported studies. This demonstrates the potential and power of the instant technique and demonstrates that, under certain conditions, PTH may actually be an inducer of pre-pro-alpha-2 chain of type I collagen mRNA synthesis.

### EXAMPLE 1. CONSTRUCTION OF cDNA LIBRARY

ROS 17/2.8 cells (ATCC CRL 11095) were grown to approximately 85% confluency in 15% FBS/F-10 media (commercially available from Gibco-BRL, Gaithersburg, MD). For control cells, 20 roller bottles were washed two times with F-10 media. 100 ml of fresh F-10, which contained 2 µg/ml of protease-free BSA (bovine serum albumin, commercially available from Gibco-BRL, Gaithersburg, MD 20877), was added and cells were incubated at 37°C for 12 hours. For treated cells, following the wash, 100 ml of fresh F-10 media containing 2 µg/ml BSA and 200 ng/ml of bovine PTH 1-34 fragment (commercially available from Bachem Bioscience, 3700 Market Street, Philadelphia, PA) was added, and cells were incubated at 37°C for 12 hours.

mRNA isolation in guanidine isothiocyanate (GNSCN) was performed in substantial accordance with the protocol of Chirgwin, et al. (1979), Isolation of Biologically Active Ribonucleic Acid from Sources Enriched in Ribonuclease, Biochemistry 18:5294-5299. Cells from a total of 20 roller bottles were collected in a final volume of 75 ml GNSCN. A Brinkman Model 10TS homogenizer (Brinkman Instruments Co.,Cantiague Road, Westbury, NY) was used to reduce the viscosity of the solution, and the mRNA was spun through CsCl gradients (Chirgwin, et al., supra) with the poly(A) section being isolated via standard protocols (see e.g. Sambrook J., E. F. Fritsch and T. Maniatis. 1989. Molecular Cloning: A Laboratory Manual, Second Edition).

Northern blots were run in substantial accordance with standard procedures such as those described in detail in Starling, et al (1990) Cancer Research 50:7634-7640.

Ten micrograms of mRNA isolated from PTH treated and control cells was provided to Stratagene (11099 North Torrey Pines Road, La Jolla, CA 92037) who created custom made directional cDNA libraries in a lambda ZAP II vector. Protocols for the construction of cDNA libraries may be found in readily available laboratory manuals in the art such as Sambrook J., E. F. Fritsch and T. Maniatis, (1989) Molecular Cloning: A Laboratory Manual, Second Edition, supra, or Current Protocols in Molecular Biology, supra. A kit for the construction of cDNA libraries in the lambda ZapII vector is commercially available from Stratagene. For the control library, the the primary number of plaques was 2.0 x 106 and the amplified titre was 2.5 x 1010 pfu/ml. For the treated library, the the primary number of plaques was 1.4 x 106 and the amplified titre was 4.5 x 1010 pfu/ml. All inserts were sized to be greater than 0.5 kb in size.

### EXAMPLE 2. PRIMARY SCREEN

Approximately 1,000 pfu of the treated Lambda Zap II cDNA library prepared in substantial accordance with the teaching of Example 1 above, was plated with E. coli XL1-blue cells (commercially available from Stratagene) on each 150 x 25mm petri dish. A total of 20 plates were used. After an eight hour incubation at 37°C, plates were briefly cooled to 4°C and DNA was lifted onto nylon filters (Amersham Hybond-N, commercially available from Amersham Corp., 2636 South Clearbrook, Arlington Heights, IL 60005). Five lifts were performed for each plate with the first lift being discarded. DNA was fixed onto the filters according to the protocol provided in the Amersham publication entitled "Blotting Protocols for Hybond^{TM} Membranes" and filters were pre-hybridized in 50mM tris (pH 7.5), 1M NaCl, 0.5% SDS, 10x Denhardt's reagent (0.2% bovine serum albumin, 0.2% Ficoll^{TM}, 0.2% Polyvinylpyrrolidone), 200µg/ml yeast RNA at 55°C for 2 hours.

Twenty-five nanograms of unlabeled cDNA, previously synthesized from treated and untreated mRNA, was labeled to approximately 1x10⁹ cpm/µg using Promega's Prime-a-gene kit (commercially available from Promega Corporation, 2800 Woods Hollow Road, Madison, WI 53711-5399) in substantial accordance with the protocol supplied by the manufacturer. The probes were directly added to the pre-hybridization solution and allowed to hybridize overnight at 55°C. Filters were briefly soaked in 6xSSPE and then washed two times in 0.1xSSPE/0.1%SDS for 30 minutes per wash at 55°C. The filters were then exposed to Kodak X-OmatAR film (commercially available from Eastman-Kodak Co., Rochester, NY) at -70°C for 3 days. Potential positive plaques, identified by differential hybridization (Sambrook, et al., supra), were cored with the tip of a sterile pasteur pipette and stored at 4° in 1 ml of 100mM NaCl, 20mM Tris (pH 7.4), 10mM MgSO4 with 2.5% chloroform.

### EXAMPLE 3. SPOT SCREENING

7 ml of NZY top agarose (0.5% NaCl, .2% MgSO₄ 7 H₂O, 0.5% Yeast Extract, 1% casein hydrolysate, 0.7% agarose) and 600µl of XL1-blue cells (Stratagene) were grown to an OD₆₀₀=0.5. The cells were mixed and poured onto a 150 x 25mm petri dish of NZY top agarose. 1µl (approximately 1,000 pfu) of each of the cored potential positive plaques was serially spotted, in quadruplicate, across the plate to form a symmetrical pattern across the plate as shown in Figure 1. Plates were incubated overnight at 37°C with plaques forming to approximately 5mm in diameter.

A single DNA lift per plate was performed. Filters were then cut directly down the middle (i.e. DNA from two spots of each clone transferred to each filter half). The left halves received PTH-treated probe while the right halves received untreated probe. Washes were done as previously described followed by an overnight X-OmatAR film exposure at -70°C. At this point, positive plaques were again cored and the SPOT protocol repeated until positive clones were plaque purified.

Figure 4 of the accompanying drawings illustrates the results of a Northern blot which confirms that the isolated plaque encodes a 4kb induced mRNA. The identity of the message as the pre-pro-alpha-2 chain of type I collagen mRNA was confirmed by DNA sequencing of the differentially regulated cDNA clone using an Applied Biosystems (Foster City, CA) Model 380A or 380B automated DNA sequencer in substantial accordance with the instructions provided by the manufacturer. Densitometric scanning of this gel demonstrated that the mRNA was induced only 2.5 fold over background. These results demonstrate that PTH is an inducer of pre-pro-alpha-2 chain of type I collagen mRNA synthesis.

## Claims

1. A method of isolating and identifying a clone containing a differentially regulated mRNA species said method comprising the steps of:
a) identifying a plaque or plaques which contain(s) a potentially differentially regulated mRNA species by conventional differential cloning techniques,
(b) obtaining a sample of a said plaque(s),
(c) serially spotting said sample of said plaque(s) across a solid media surface,
(d) lifting, and fixing DNA from said solid media surface onto a transfer membrane,
(e) dividing said transfer membrane such that each section contains a sample of DNA from each serially spotted plaque,
(f) exposing one section of said transfer membrane to treated probes and the other section of said transfer membrane to untreated probes, and
(g) identifying induced or repressed mRNA by the pattern of differential hybridization.

2. A method of the present invention comprises the steps of:
a) identifying a colony or colonies which contain(s) a potentially differentially regulated mRNA species by conventional differential cloning techniques,
(b) obtaining a sample of a said colony(s),
(c) serially spotting said sample of said colony(s) across a solid media surface,
(d) lifting and fixing DNA from said solid media surface onto a transfer membrane,
(e) dividing said transfer membrane such that each section contains DNA from each serially spotted colony,
(f) exposing one section of said transfer membrane to treated probes and the other section of said transfer membrane to untreated probes, and
(g) identifying induced or repressed mRNA by the pattern of differential hybridization.

3. A method to confirm the identify of a mRNA species as a differentially regulated mRNA, said method comprising the method of Claim 1 further comprising the steps of:
(h) removing the treated or untreated probe from individual sections,
(i) rehybridizing the section previously exposed with the treated probe with the untreated probe and rehybridizing the section previously exposed to untreated probe with the treated probe, and
(j) evaluating these sections for a reversal of the previous hybridization pattern.

4. A method to confirm the identify of a mRNA species as a differentially regulated mRNA, said method comprising the method of Claim 2 further comprising the steps of:
(h) removing the treated or untreated probe from individual sections,
(i) rehybridizing the section previously exposed with the treated probe with the with the untreated probe and rehybridizing the section previously exposed to untreated probe with the treated probe, and
(j) evaluating these sections for a reversal of the previous hybridization pattern.

5. A kit comprising materials to practice the method of claim 1.

6. A kit comprising materials to practice the method of claim 2.
